# FASCICULE DE BREVET EUROPEEN

(11) **EP 3 566 047 B1**
(45) Date de publication et mention de la délivrance du brevet: **15.05.2024**
(21) Numéro de dépôt: 17821598.4
(22) Date de dépôt: 19.12.2017
(51) Int. Cl.: H04M 1/21, H04R 1/02, G01N 33/00, H04R 1/08

(54) **MICROPHONE COMPRENANT DES MOYENS DE MESURE DE GAZ ET PROCEDE DE MESURE DE GAZ A L'AIDE D'UN TEL MICROPHONE**
MIKROFON MIT GASMESSMITTELN UND VERFAHREN ZUR GASMESSUNG MIT SOLCH EINEM MIKROFON
MICROPHONE COMPRISING GAS-MEASURING MEANS AND METHOD FOR MEASURING GAS BY MEANS OF SUCH A MICROPHONE

(30) Priorité: 04.01.2017 FR 1750068
(43) Date de publication de la demande: 13.11.2019
(73) Titulaire: ELLONA, 31400 Toulouse (FR)
(72) Inventeur: MIFSUD, Jean-Christophe, 82400 Goudourville (FR)
(74) Mandataire: Argyma
(86) Numéro de dépôt international: PCT/EP2017/083521
(87) Numéro de publication internationale: WO 2018/127403

(56) Documents cités:
- EP-A1- 3 187 865
- WO-A1-03/031968
- CH-A5- 691 197
- JP-A- 2002 039 981
- US-A1- 2003 094 047
- TZENG TE-HSUEN ET AL: "A Portable Micro Gas Chromatography System for Lung Cancer Associated Volatile Organic Compound Detection", IEEE JOURNAL OF SOLID-STATE CIRCUITS, IEEE, USA, vol. 51, no. 1, 1 January 2016 (2016-01-01), pages 259-272, XP011596423, ISSN: 0018-9200, DOI: 10.1109/JSSC.2015.2489839 [retrieved on 2015-12-30]

## Description

### DOMAINE TECHNIQUE GENERAL ET ART ANTERIEUR

La présente invention concerne le domaine de la mesure de l'air et, plus particulièrement, de l'haleine d'une personne afin de mesurer le taux d'alcoolémie, le niveau d'hydratation et/ou l'odeur de l'haleine d'une personne.

Aujourd'hui, pour mesurer le taux d'alcoolémie dans l'haleine d'un utilisateur, un éthylotest comprend un capteur de gaz relié à un embout par lequel l'utilisateur souffle de l'air. Le capteur de gaz mesure ainsi la quantité d'alcool présent dans l'air soufflé par l'utilisateur. L'éthylotest comporte en outre un débitmètre pour mesurer le débit d'air soufflé par l'utilisateur et ainsi en déduire le taux d'alcoolémie.

Afin de faciliter l'utilisation de l'éthylotest, il est aujourd'hui connu de relier l'éthylotest à un téléphone intelligent, dit « smartphone », de manière sans fil ou filaire. L'utilisateur peut ainsi lire le taux d'alcoolémie mesuré par l'éthylotest sur l'écran du téléphone.

Cependant, un tel éthylotest présente des inconvénients. En effet, l'éthylotest est un élément indépendant du téléphone que l'utilisateur doit donc toujours porter sur lui pour mesurer son haleine, ce qui est encombrant et augmente le risque d'oubli ou de perte.

Une solution serait d'intégrer l'éthylotest directement dans un téléphone intelligent exemplifiée dans le document JP2002 039981 A. Cependant, l'intégration d'un capteur de gaz et d'un débitmètre nécessite de prévoir un orifice dans le téléphone par lequel l'utilisateur souffle afin de faire passer l'air à mesurer. Or, de la poussière et des liquides peuvent s'infiltrer à l'intérieur du téléphone par cet orifice, ce qui peut l'endommager et représente donc un inconvénient majeur. De plus, l'intégration d'un capteur de gaz et d'un débitmètre dans un téléphone intelligent, dans lequel l'espace disponible est faible, est complexe et augmente son coût.

Dans un autre domaine, on connaît par le document US 2003/094047 A1, un microphone comprenant un capteur permettant de mesurer la pression de l'air au niveau du microphone. Cependant, un tel capteur ne permet pas de mesurer le taux d'un gaz, et en particulier le taux d'alcoolémie dans l'haleine d'un utilisateur.

Il existe donc un besoin pour un capteur de gaz permettant de mesurer de manière fiable et efficace un taux d'alcoolémie et, plus généralement la quantité de plusieurs gaz, et qui puisse être intégré de manière pratique et à moindre coût dans un appareil électronique, notamment, un téléphone.

### PRESENTATION GENERALE DE L'INVENTION

A cet effet, l'invention concerne un microphone comprenant au moins une membrane rigide perforée et au moins une membrane flexible placée à distance de la membrane rigide de manière à former une enceinte entre la membrane rigide et ladite membrane flexible, la membrane flexible étant adaptée pour être déplacée par une vibration sonore de l'air extérieur au microphone via la membrane rigide perforée.

L'invention est remarquable en ce que le microphone comprend des moyens de mesure de la quantité d'au moins un gaz, lesdits moyens de mesure étant montés dans l'enceinte afin de mesurer au moins un gaz présent dans ladite enceinte.

Grâce à l'invention, il est possible de mesurer de manière fiable et efficace la quantité d'un gaz au moyen d'un microphone. De manière avantageuse, le microphone remplit ainsi la double fonction de mesure de sons et de mesure de gaz. Le microphone existant est adapté afin d'intégrer des moyens de mesure. Un tel microphone peut être monté dans un appareil électronique en lieu et place d'un microphone existant afin de permettre audit appareil de mesurer des gaz. Aussi, il n'est pas nécessaire de prévoir un orifice supplémentaire dans l'appareil électronique, ce qui limite les coûts.

De préférence, les moyens de mesure sont montés sur la membrane flexible, notamment par collage, afin d'être en contact avec l'air présent dans l'enceinte. De plus, le montage par collage permet d'assembler aisément les moyens de mesure dans le microphone lors de sa fabrication. Un tel positionnement permet d'éviter de modifier la membrane perforée et/ou d'obturer des performations. De manière avantageuse, l'ajout des moyens de mesure n'affecte que faiblement la flexibilité de la membrane flexible. De préférence, les moyens de mesure sont montés exclusivement sur la membrane flexible.

De manière alternative, les moyens de mesure sont montés sur la membrane rigide, notamment par gravure sur une membrane du microphone, ce qui permet de limiter le nombre d'éléments du microphone et ainsi réduire les coûts de fabrication et d'assemblage. De préférence, les moyens de mesure sont montés exclusivement sur la membrane rigide.

Selon l'invention, les moyens de mesure comprennent au moins un chromatographe gazeux permettant de mesurer séparément la quantité des molécules de différents gaz.

L'invention concerne également un appareil électronique, en particulier un téléphone, comprenant un microphone tel que décrit précédemment, adapté, d'une part, pour mesurer des bruits et des sons et, d'autre part, pour mesurer au moins un gaz dans l'air extérieur à l'appareil électronique. Ainsi, les moyens de mesure peuvent mesurer les gaz dans l'air expiré par l'utilisateur lorsque ce dernier utilise l'appareil électronique, notamment un téléphone. L'invention vise en outre un procédé de mesure à l'aide d'un microphone tel que décrit précédemment, du taux d'au moins un gaz dans l'air expiré par un utilisateur, ledit procédé comprenant :
- Une étape d'expiration d'air par un utilisateur dans ledit microphone,
- Une étape de mesure par les moyens de mesure dudit microphone de la quantité d'au moins un gaz dans l'air expiré,
- Une étape de détermination par le microphone du volume d'air expiré par l'utilisateur, et
- Une étape de détermination du taux du gaz mesuré dans l'air expiré à partir de ladite quantité mesurée du gaz et dudit volume déterminé d'air expiré.

Ainsi, le microphone permet de mesurer le débit d'air au niveau des moyens de mesure et ne nécessite pas un débitmètre supplémentaire, ce qui limite le nombre d'élément dans le microphone et son coût. De plus, la mesure étant effectuée dans l'air expiré par l'utilisateur, ce geste est naturel, ce qui rend l'utilisation du microphone aisée pour l'utilisateur. Avantageusement, le volume d'air expiré est déterminé en mesurant la pression exercée par l'air expiré sur la membrane flexible. Le volume d'air expiré est ainsi mesuré de manière pratique en mesurant des paramètres caractéristiques à un microphone. L'intégration des moyens de mesure dans le microphone présente donc des synergies.

De préférence, le microphone étant compris dans un téléphone, l'étape de mesure de la quantité du gaz dans l'air expiré est réalisée lors d'un appel téléphonique de l'utilisateur. Ainsi, une mesure de l'haleine peut être réalisée de manière automatique sans action spécifique de l'utilisateur. Une mesure de l'haleine est ainsi non-intrusive et peut-être réalisée de manière fréquente.

### PRESENTATION DES FIGURES

L'invention sera mieux comprise à la lecture de la description qui va suivre, donnée uniquement à titre d'exemple, et se référant aux dessins annexés sur lesquels :
- La figure 1 est une vue schématique d'un téléphone mobile comprenant un microphone selon l'invention,
- la figure 2 est une vue schématique en coupe d'une première forme de réalisation du microphone ne faisant pas partie de l'invention,
- la figure 3 est une vue schématique des moyens de mesure du microphone de la figure 2,
- la figure 4 est une vue schématique en coupe d'une deuxième forme de réalisation du microphone selon l'invention, et
- la figure 5 est une vue schématique des moyens de mesure du microphone de la figure 4.

Il faut noter que les figures exposent l'invention de manière détaillée pour mettre en oeuvre l'invention, lesdites figures pouvant bien entendu servir à mieux définir l'invention le cas échéant.

### DESCRIPTION D'UN OU PLUSIEURS MODES DE REALISATION ET DE MISE EN OEUVRE

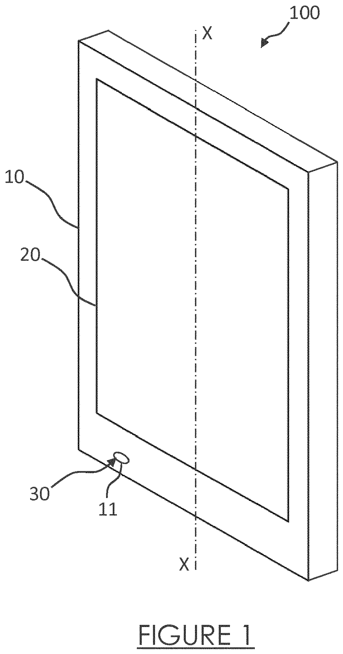

En référence à la figure 1, il est représenté de manière schématique un téléphone mobile 100 du type intelligent, également désigné smartphone en langue anglaise. Le téléphone mobile 100 comprend un corps 10, un écran 20 d'affichage monté sur le corps 10, un microphone 30 et un système électronique (non représenté) adapté pour contrôler les fonctions du téléphone.

Dans cet exemple, le corps 10 présente une forme sensiblement parallélépipédique s'étendant longitudinalement selon un axe X-X de manière à définir une partie inférieure dans laquelle est monté le microphone 30 et une partie supérieure dans laquelle est monté un haut-parleur (non représenté). Le corps 10 permet de monter et de protéger différents éléments du téléphone 100, tels que le système électronique, une batterie, etc. Le corps 10 comprend également deux grandes faces sur l'une desquelles est monté l'écran 20.

Le corps 10 comprend en outre un orifice 11 dans lequel est monté le microphone 30. De préférence, l'orifice 11 est placé au niveau de la partie inférieure du téléphone 100 de manière à être à proximité de la bouche de l'utilisateur lorsque l'utilisateur utilise le téléphone 100 pour passer un appel. L'orifice 11 permet au microphone 30 d'être en contact avec l'air présent à l'extérieur du téléphone 100 afin de recevoir les sons, tels que la voix de l'utilisateur lors d'un appel, se propageant à travers l'air. Un tel téléphone est connu de l'homme du métier et ne sera pas présenté plus en détails.

Le téléphone 100 comprend un microphone 30 selon l'invention qui va maintenant être décrit.

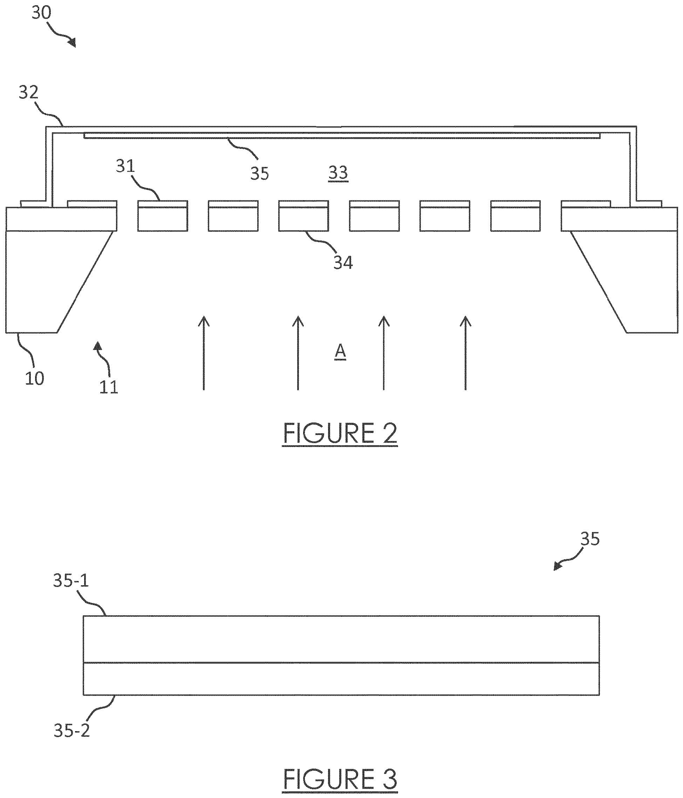

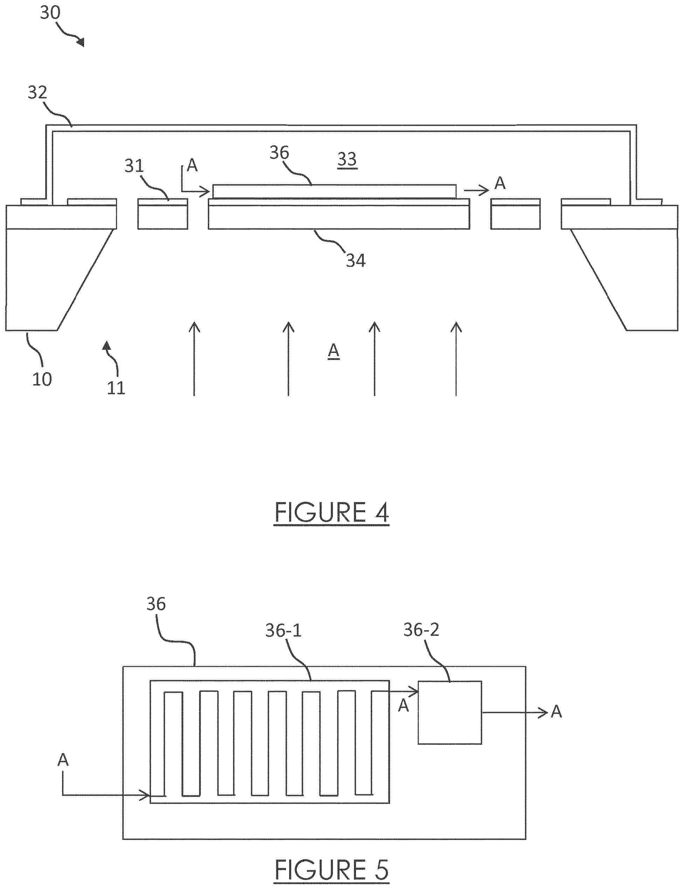

En référence aux figures 2 et 4, il est représenté un microphone 30 de type MEMS, pour « MicroElectrical-Mechanical System » en langue anglaise qui signifie microsystème électromécanique. Le microphone 30 comprend une membrane rigide 31 et une membrane flexible 32 placée à distance de la membrane rigide 31 de manière à former une enceinte 33.

La membrane rigide 31 est perforée de manière à ce que l'enceinte 33 soit reliée fluidiquement à l'air extérieur au téléphone A. Ainsi, un son se propageant dans l'air extérieur A se propage à l'intérieur de l'enceinte 33 comme illustré aux figures 2 et 4.

La membrane rigide 31 peut être réalisée en silicium, en céramique ou en métal par exemple. La membrane flexible 32 est une membrane fine réalisée en un matériau composite par exemple, adapté pour être déformé lorsque la pression exercée par l'air A sur la membrane flexible 32 varie. De préférence, la membrane flexible 32 comprend une partie périphérique cylindrique s'étendant orthogonalement à la membrane rigide 31 et une partie plane parallèle à la membrane rigide 31 comme illustré à la figure 2.

Avantageusement, le microphone 30 selon l'invention peut être monté en lieu et place d'un microphone selon l'art antérieur. Le microphone 30 selon l'invention est relié électriquement au système électronique de manière analogue à l'art antérieur, ce qui rend aisé son intégration dans le téléphone 100. Ainsi, le montage du microphone 30 ne nécessite pas ou peu de modification du téléphone 100, seuls les moyens de mesure doivent être reliés au système électronique du téléphone 100.

De manière connue, lorsqu'un son se propage dans l'air A jusqu'au microphone 30, la les ondes sonores modifient la pression de l'air A sur la membrane flexible 32. Aussi, pour mesurer un son se propageant dans l'air A, le microphone 30 mesure la variation de la distance entre la membrane rigide 31 et la membrane flexible 32 due aux ondes sonores.

Pour ce faire, le microphone 30 comporte un circuit imprimé 34 relié à la membrane rigide 31 et à la membrane flexible 32 de manière à former un condensateur dont la capacité varie selon la distance entre la membrane rigide 31 et la membrane flexible 32. Ainsi, le microphone 30 transforme un son en signal électrique représentatif dudit son. Le microphone 30 est alors adapté pour envoyer le signal électrique représentatif du son au système électronique du téléphone 100 afin d'être exploité. Le fonctionnement d'un tel microphone étant connu, il ne sera pas décrit plus en détail.

Le microphone 30 selon l'invention comprend en outre des moyens de mesure adaptés pour mesurer au moins un gaz présent dans l'air A, tel que de l'éthanol, de l'acétone, etc. Selon l'invention, les moyens de mesure sont montés dans l'enceinte 33 du microphone 30 afin d'être en contact avec l'air A. Ainsi, les moyens de mesure ne nécessitent aucun orifice supplémentaire dans le téléphone 100. De plus, les moyens de mesure sont protégés dans l'enceinte 33, notamment des poussières, des chocs, etc.

Selon une première forme de réalisation ne faisant pas partie de l'invention et en référence aux figures 2 et 3, les moyens de mesure comprennent au moins un capteur oxydo-métallique 35, également désigné MOS. Un tel capteur MOS 35 comprend une couche sensible 35-1 à au moins un gaz et une couche chauffante 35-2 montée sur ladite couche sensible 35-1.

La couche sensible 35-1 est en contact avec l'air A et est adaptée pour absorber des molécules du gaz à mesurer. L'absorption de ces molécules fait varier la conductivité électrique à travers la couche sensible 35-1 grâce à une réaction d'oxydo-réduction, ce qui permet de déterminer la quantité dudit gaz présent dans l'air A en contact avec la couche sensible 35-1. Dans ce cas, la quantité de gaz est mesurée à partir de la variation de la résistance dR par rapport à la résistance initiale R₀ du capteur MOS 35. Autrement dit, la quantité de gaz mesurée est égale au rapport dR/R₀.

La couche sensible 35-1 comprend un oxyde permettant la réaction d'oxydo-réduction avec le gaz, par exemple, des molécules de dioxyde d'étain (SnO2), de dioxyde de titanium (TiO2), de trioxyde de tungstène (WO3) et/ou de l'oxyde de Niobium (Ne2O5) avec des dopages d'éléments de Platine (Pt), d'or (Au), de Germanium (Ge) et/ou de Palladium (Pd). Les éléments chimiques qui sont mélangés avec des molécules permettent de doper ces dernières afin d'optimiser leur fonction d'absorption de molécules du gaz à mesurer.

La couche chauffante 35-2 est commandée, de préférence par le système électronique du téléphone 100, afin de modifier la température de la couche sensible 35-1, ce qui permet à la couche sensible 35-1 d'absorber différents gaz: chaque température de la couche sensible 35-1 permettant de mesurer la quantité d'un gaz. Grâce à la couche chauffante 35-2, une seule couche sensible 35-1 permet de mesurer différents gaz, ce qui permet de limiter la consommation en énergie électrique et le coût du capteur MOS 35. La couche chauffante 35-2 comprend dans cet exemple un substrat, tel que de la céramique ou du silicium, et des éléments, tels que de l'or, adaptés pour dégager de la chaleur lorsqu'ils sont traversés par un courant électrique. Le fonctionnement d'un capteur MOS 35 étant connu, il ne sera pas décrit plus en détail.

Le capteur MOS 35 peut être monté sur la membrane rigide 31 ou bien sur toute partie de la membrane flexible 32, notamment la paroi et la partie plane, du fait de sa flexibilité compatible avec la flexibilité de la membrane flexible 32. Ainsi, la couche sensible 35-1 est en contact avec l'air A présent dans l'enceinte 33. Le capteur MOS 35 peut être monté dans l'enceinte 33, par exemple par collage de la couche chauffante 35-2 sur l'enceinte 33 afin que la couche sensible 35-1 soit en contact avec l'air A.

Les moyens de mesure pourraient également comprendre plus d'un capteur MOS 35. Par exemple, des capteurs MOS 35 dont l'oxyde est différent afin de détecter différents éléments du gaz à mesurer et ainsi optimiser cette mesure. Les capteurs MOS 35 peuvent également être placés à différents endroits: au moins un premier capteur MOS 35 pouvant être monté sur la membrane flexible 32 et au moins un deuxième capteur MOS 35 pouvant être monté sur la membrane rigide 31. Ceci permet notamment d'effectuer deux mesures de l'air A décalées dans le temps afin de déterminer la différence d'inertie entre différentes molécules de l'air A (plus une molécule présente dans l'air A présente des dimensions importantes, plus elle mettra de temps pour être déplacée entre les deux capteurs), ce qui permet d'optimiser la détection de gaz dans l'air A grâce à ce paramètre supplémentaire. L'utilisation de deux capteurs MOS 35 peut également permettre d'utiliser un premier capteur MOS 35 comme un filtre, notamment en augmentant sa température afin de brûler certains gaz, tel que l'éthanol, et permettre ainsi au deuxième capteur MOS 35 de mesurer les différents gaz de l'air sans l'éthanol, ce qui optimise la mesure.

Alternativement, la membrane flexible 32 est formée par la couche sensible 35-1 du capteur MOS 35. Autrement dit, la couche sensible 35-1 du capteur MOS 35 est la membrane flexible 32 du microphone 30 afin de minimiser le nombre d'éléments dans le microphone 30 et ainsi d'en réduire les coûts. Ceci permet également d'optimiser les dimensions de la couche sensible 35-1 et ainsi d'améliorer la mesure de gaz.

Les moyens de mesure de gaz peuvent mettre en oeuvre des technologies de détection sélectives, par exemple basées sur les nanotubes en carbone, ou non sélectives, par exemple la technologie MOX.

Selon l'invention et en référence aux figures 4 et 5, les moyens de mesure comprennent au moins un chromatographe gazeux 36. Un tel chromatographe gazeux 36 permet avantageusement de mesurer quantitativement une pluralité de gaz en simultanée. De préférence, le chromatographe gazeux 36 est monté sur la membrane rigide 31, par exemple par gravure.

Le chromatographe gazeux 36 comprend une colonne 36-1 et un détecteur 36-2 de gaz à la sortie de la colonne 36-1.

L'air A de l'enceinte 33 est injecté dans la colonne 36-1 lors de l'expiration de l'utilisateur. L'air A traverse alors la colonne 36-1 comprenant un solide absorbant, également désigné phase stationnaire. En traversant la phase stationnaire, les gaz compris dans l'air A sont séparés du fait de l'affinité différente de chaque gaz avec la phase stationnaire ce qui entraîne une vitesse différente de propagation de chaque gaz à travers la colonne 36-1. A la sortie de la colonne 36-1, les gaz compris dans l'air A étant séparés, la quantité de chaque gaz compris dans l'air A est alors mesurée par le détecteur de gaz 36-2. Un tel détecteur de gaz 36-2 peut notamment être un détecteur à thermocouple gravé. Le chromatographe gazeux 36 permet ainsi de mesurer la quantité de plusieurs gaz présents dans l'air A de manière indépendante. Le fonctionnement d'une telle chromatographie gazeuse étant connue, elle ne sera pas décrite plus en détail. Avantageusement, la colonne 36-1 du chromatographe gazeux 36 est gravée sur la membrane rigide du microphone 30 afin de rendre sa fabrication et son assemblage aisés.

Afin de mesurer la quantité d'un gaz présent en faible quantité, le chromatographe gazeux 36 peut également comprendre un pré-concentrateur en entrée de la colonne 36-1. Un tel pré-concentrateur peut notamment être du type à charbon actif ou du tenax et est adapté pour capter les molécules d'un gaz à mesurer puis à les libérer en plus grande quantité à une certaine température. Le fonctionnement d'un pré-concentrateur étant connu, il ne sera pas décrit plus en détail.

Les moyens de mesure sont reliés électriquement au circuit imprimé 34 du microphone 30 de manière à ce que celui-ci collecte les données mesurées. De manière avantageuse, le circuit imprimé 34 comporte plus de connecteurs qu'un circuit imprimé selon l'art antérieur pour communiquer avec le système électronique du téléphone. Autrement dit, il est uniquement nécessaire d'adapter le nombre de connecteurs du système électronique pour accueillir le microphone selon l'invention.

Il va maintenant être décrit un exemple de mise en oeuvre du procédé de mesure d'un gaz selon l'invention à l'aide d'un microphone 30.

Pour mesurer son haleine, l'utilisateur souffle de l'air A vers le microphone 30. L'air A rentre alors dans l'enceinte 33 jusqu'aux moyens de mesure qui mesurent alors la quantité d'au moins un gaz représentatif de l'haleine de l'utilisateur dans l'air A. Dans cet exemple, la quantité mesurée est de l'ordre de 0,02 grammes Les moyens de mesure envoient alors cette mesure au système électronique du téléphone 100 qui peut effectuer directement des calculs ou bien les envoyer à un calculateur distant par un réseau de communication afin que les calculs soient effectués à distance.

De manière simultanée, le microphone 30 mesure la pression qu'exerce le souffle d'air A sur la membrane flexible 32 en mesurant la variation de la distance entre la membrane rigide 31 et la membrane flexible 32. Dans cet exemple, la pression est déterminée à partir de la mesure de la variation de la pression au sein de l'enceinte 33 lorsque l'utilisateur souffle. Ici, la variation mesurée est de l'ordre de 10 hectopascals. Cette pression est alors envoyée au système électronique du téléphone qui détermine le débit d'air A soufflé par l'utilisateur à partir de la pression mesurée et de la surface de la membrane flexible 31. Dans cet exemple toujours, pour une surface de 4 mm², le débit est alors égal à 0,2 Us. Puis, le système électronique détermine le volume d'air A soufflé par l'utilisateur à partir du débit déterminé et du temps pendant lequel l'utilisateur souffle de l'air A. Dans cet exemple, pour une durée de 1 seconde, le volume d'air A est égal à 0,2 L.

Puis, le système électronique du téléphone 100 calcul le taux du gaz dans l'air A à partir de la quantité de ce gaz mesurée par les moyens de mesure et le volume déterminé d'air A que l'utilisateur à souffle. Dans cet exemple, pour une quantité de 0,2 mg de gaz et un volume d'air A de 1 L, le taux du gaz dans l'air A est de 0,02 mg/L.

Avantageusement, la mesure de l'haleine de l'utilisateur peut être réalisée lorsque ce dernier passe un appel. Pour cela, l'utilisateur saisit le téléphone 100 verticalement et place le téléphone contre son oreille. L'orifice 11 est alors à proximité de la bouche de l'utilisateur de sorte que l'air A entrant dans l'enceinte 33 soit l'haleine que l'utilisateur expire en parlant. Les moyens de mesure présents dans l'enceinte 33 mesurent alors la quantité d'au moins un gaz représentatif de l'haleine de l'utilisateur.

En parlant, l'utilisateur émet également des ondes sonores qui se propagent dans l'air A. Ces ondes sonores passent alors à travers la membrane rigide 31 perforée du microphone 30 afin d'entrer dans l'enceinte 33. Les ondes sonores font alors varier la pression au niveau de la membrane flexible 32, ce qui entraîne une modification de la valeur de la capacité du condensateur formé par la membrane rigide 31 et la membrane flexible 32. Le microphone 30 mesure alors la pression exercée par l'air A sur la membrane flexible et la durée pendant laquelle cette pression est exercée afin de déterminer le débit d'air A expiré par l'utilisateur. La variation de la capacité permet en outre de déterminer les ondes sonores émises afin de les transmettre à l'interlocuteur de l'utilisateur.

A partir de la quantité mesurée du gaz représentatif de l'haleine et du débit de l'air A expiré, le microphone détermine le taux dudit gaz représentatif de l'haleine dans l'air A expiré.

Grâce au microphone selon l'invention, il est possible de mesurer l'haleine d'un utilisateur d'un téléphone sans trous supplémentaires dans le téléphone ce qui permet de protéger le téléphone. De plus, l'intégration de moyens de mesure dans le microphone permet de minimiser l'encombrement de tels moyens de mesure dans un téléphone ainsi que son coût. En outre, une telle intégration permet de limiter l'infiltration des poussières et des liquides.

Avantageusement, le microphone ne comporte qu'une unique ouverture pour permettre l'entrée et la sortie d'air.

De manière avantageuse, les moyens de mesure de gaz permettent d'utiliser des moyens techniques propres au microphone, notamment, des moyens de convertisseurs de signaux numériques-analogiques. Comme les moyens techniques sont mutualisés, le microphone obtenu possède un coût inférieur à un ensemble comprenant un microphone indépendant et un capteur de gaz indépendant.

Un tel microphone peut être utilisé pour mesurer le taux d'alcoolémie d'une personne, pour détecter une contamination de l'air ambiant, pour analyser l'haleine d'une personne dans le cadre d'un diagnostic médical ou bien pour analyser la fraîcheur d'aliments par exemple. Grâce au microphone selon l'invention, il est possible de créer des alertes en cas de mesures anormales dans l'air ambiant ou dans l'haleine de l'utilisateur lors de ses appels téléphoniques. Ceci permet notamment d'effectuer un suivi en permanence de biomarqueurs présents dans l'haleine et qui peuvent être liés à l'état de santé de l'utilisateur. Ces biomarqueurs peuvent notamment être des marqueurs de consommation de la graisse pendant des exercices physiques, des marqueurs du diabète, du cancer du poumon, de la tuberculose, etc.

Il a été présenté la mesure d'un gaz lors d'un appel, mais il va de soi que les moyens de mesure pourraient mesurer un gaz de manière continue, notamment dans le cas de la détection d'une contamination de l'air ambiant afin d'alerter l'utilisateur. Les moyens de mesure pourraient également effectuer une mesure à la demande de l'utilisateur, notamment dans le cas de l'analyse de la fraîcheur d'un aliment.

Le microphone 30 selon l'invention étant relié au système électronique du téléphone 100 de manière similaire à un microphone selon l'art antérieur, il est possible de remplacer un microphone existant d'un téléphone par un nouveau microphone selon l'invention afin d'obtenir de nouvelles fonctions, telles que la mesure de l'haleine de l'utilisateur.

Il a été présenté un microphone 30 monté dans un téléphone 100. Mais il va de soi que le microphone 30 pourrait être monté dans tout autre appareil électronique, notamment un casque de musique, des écouteurs, une montre, un interphone, un véhicule, etc.

## Revendications

1. Microphone (30) comprenant au moins une membrane rigide (31) perforée et au moins une membrane flexible (32) placée à distance de la membrane rigide (31) de manière à former une enceinte (33) entre la membrane rigide (31) et la membrane flexible (32), la membrane flexible (32) étant adaptée pour être déplacée par une vibration sonore de l'air extérieur au microphone via la membrane rigide perforée (31), **ledit microphone (30) étant caractérisé en ce qu'**il comprend des moyens de mesure (35, 36) de la quantité d'au moins un gaz expiré par un utilisateur, lesdits moyens de mesure étant montés dans l'enceinte (33) afin de mesurer au moins un gaz présent dans ladite enceinte (33), les moyens de mesure comprenant au moins un chromatographe gazeux (36).

2. Microphone (30) selon la revendication 1, dans lequel les moyens de mesure (35) sont montés sur la membrane flexible (32).

3. Microphone (30) selon la revendication 1, dans lequel les moyens de mesure (36) sont montés sur la membrane rigide (31).

4. Appareil électronique, en particulier un téléphone (100), comprenant un microphone (30) selon l'une des revendications précédentes, adapté, d'une part, pour mesurer des bruits et des sons et, d'autre part, pour mesurer au moins un gaz dans l'air (A) extérieur à l'appareil électronique.

5. Procédé de mesure à l'aide d'un microphone (30) selon l'une des revendications 1 à 3, du taux d'au moins un gaz dans l'air (A) expiré par un utilisateur, ledit procédé comprenant :
- Une étape d'expiration d'air (A) par un utilisateur dans ledit microphone (30),
- Une étape de mesure par les moyens de mesure (35, 36) dudit microphone (30) de la quantité d'au moins un gaz dans l'air (A) expiré,
- Une étape de détermination par le microphone (30) du volume d'air (A) expiré par l'utilisateur, et
- Une étape de détermination du taux du gaz mesuré dans l'air (A) expiré à partir de ladite quantité mesurée du gaz et dudit volume déterminé d'air (A) expiré.

6. Procédé selon la revendication précédente, dans lequel le volume d'air (A) expiré est déterminé en mesurant la pression exercée par l'air (A) expiré sur la membrane flexible (32).

7. Procédé selon l'une des revendications 5 à 6, dans lequel, le microphone (30) étant compris dans un téléphone (100), l'étape de mesure de la quantité du gaz dans l'air (A) expiré est effectuée lors d'un appel téléphonique de l'utilisateur afin de mesurer le taux du gaz dans l'air (A) expiré par l'utilisateur durant ledit appel.

## Patentansprüche

1. Mikrofon (30), umfassend mindestens eine perforierte starre Membran (31) und mindestens eine flexible Membran (32), die von der starren Membran (31) beabstandet platziert ist, so dass zwischen der starren Membran (31) und der flexiblen Membran (32) ein Raum (33) gebildet wird, wobei die flexible Membran (32) geeignet ist, durch eine Schallvibration der Luft außerhalb des Mikrofons über die perforierte starre Membran (31) bewegt zu werden, wobei das Mikrofon (30) **dadurch gekennzeichnet ist, dass** es Messmittel (35, 36) der Menge mindestens eines von einem Benutzer ausgeatmeten Gases umfasst, wobei die Messmittel in dem Raum (33) angebracht sind, um mindestens ein in dem Raum (33) vorhandenes Gas zu messen, wobei die Messmittel mindestens einen Gaschromatographen (36) umfassen.

2. Mikrofon (30) nach Anspruch 1, wobei die Messmittel (35) auf der flexiblen Membran (32) angebracht sind.

3. Mikrofon (30) nach Anspruch 1, wobei die Messmittel (36) auf der starren Membran (31) angebracht sind.

4. Elektronisches Gerät, vor allem ein Telefon (100), umfassend ein Mikrofon (30) nach einem der vorangehenden Ansprüche, das einerseits angepasst ist, um Geräusche und Töne zu messen, und andererseits, um ein Gas in der Luft (A) außerhalb des elektronischen Geräts zu messen.

5. Messverfahren mit Hilfe eines Mikrofons (30) nach einem der Ansprüche 1 bis 3 des Gehalts mindestens eines von einem Benutzer ausgeatmeten Gases in der Luft (A), wobei das Verfahren umfasst:
- einen Schritt des Ausatmens von Luft (A) durch einen Benutzer in das Mikrofon (30),
- einen Schritt des Messens der Menge mindestens eines ausgeatmeten Gases in der Luft (A) durch die Messmittel (35, 36) des Mikrofons (30),
- einen Schritt des Bestimmens des Volumens der von dem Benutzer ausgeatmeten Luft (A) durch das Mikrofon (30), und
- einen Schritt des Bestimmens des Gehalts des in der Luft (A) ausgeatmeten Gases ausgehend von der gemessenen Menge des Gases und vom bestimmten Volumen der ausgeatmeten Luft (A).

6. Verfahren nach vorangehendem Anspruch, wobei das Volumen der ausgeatmeten Luft (A) durch Messen des Drucks bestimmt wird, der von der ausgeatmeten Luft (A) auf die flexible Membran (32) ausgeübt wird.

7. Verfahren nach einem der Ansprüche 5 bis 6, wobei, wobei das Mikrofon (30) in einem Telefon (100) enthalten ist, der Schritt des Messens der Menge des Gases in der ausgeatmeten Luft (A) bei einem Telefonanruf des Benutzers durchgeführt wird, um den Gehalt des Gases in der von dem Benutzer ausgeatmeten Luft (A) während des Anrufs zu messen.

## Claims

1. Microphone (30) comprising at least one perforated rigid membrane (31) and at least one flexible membrane (32) placed at a distance from the rigid membrane (31) so as to form an enclosure (33) between the rigid membrane (31) and the flexible membrane (32), the flexible membrane (32) being suited to being displaced by a sound vibration of air outside of the microphone via the perforated rigid membrane (31), said microphone (30) being **characterised in that** it comprises means for measuring (35, 36) the quantity of at least one gas breathed out by a user, said means for measuring being mounted in the enclosure (33) in order to measure at least one gas present in said enclosure (33), the means for measuring comprising at least one gas chromatograph (36).

2. Microphone (30) according to claim 1, in which the means for measuring (35) are mounted on the flexible membrane (32).

3. Microphone according to claim 1, in which the means for measuring (36) are mounted on the rigid membrane (31).

4. Electronic device, in particular a telephone (100), comprising a microphone (30) according to one the preceding claims, suited, on the one hand, to measuring noises and sounds and, on the other hand, to measuring at least one gas in the air (A) outside of the electronic device.

5. Method for measuring, using a microphone (30) according to one of claims 1 to 3, the level of at least one gas in the air (A) breathed out by a user, said method comprising:
- A step of breathing out air (A) by a user into said microphone (30),
- A step of measuring, by the means for measuring (35, 36) of said microphone (30), the quantity of at least one gas in the air (A) breathed out,
- A step of determining by the microphone (30) the volume of air (A) breathed out by the user, and
- A step of determining the level of the gas measured in the air (A) breathed out from said measured quantity of the gas and said determined volume of air (A) breathed out.

6. Method according to the preceding claim, in which the volume of air (A) breathed out is determined by measuring the pressure exerted by the air (A) breathed out on the flexible membrane (32).

7. Method according to one of claims 5 to 6, in which, the microphone (30) being comprised in a telephone (100), the step of measuring the quantity of gas in the air (A) breathed out is carried out during a telephone call of the user in order to measure the level of gas in the air (A) breathed out by the user during said call.
